# EUROPEAN PATENT SPECIFICATION

(11) **EP 1 166 800 B1**
(45) Date of publication and mention of the grant of the patent: **22.03.2006**
(21) Application number: 00830454.5
(22) Date of filing: 28.06.2000
(51) Int. Cl.: A61K 45/06, A61K 31/733, A61K 31/702, A61K 47/36, A61K 47/26, A61K 9/08

(54) **Pharmaceutical compositions containing antibiotics and fructan mixtures**
Pharmazeutische Zusammensetzungen von Antibiotika und Fruktan-Gemische
Compositions pharmaceutiques contenant des antibiotiques et des mélanges de fructosane

(43) Date of publication of application: 02.01.2002
(73) Proprietor: B.S.D. BIO SCIENCE DEVELOPMENT SNC Di OMINI C. & ZUCCARI G., 20060 Bussero (Milano) (IT)
(72) Inventor: Corradini, Claudio, 00189 Roma (IT); Omini, Claudio, 20060 Bussero (MI) (IT); Bonesso, Giampiero, Monte Carlo (MC); Tortora, Diomede, 95126 Catania (IT); Pisani, Giorgio, 20100 Milano (IT); Zuccari, Giuseppe, 26866 S. Angelo Lodig (IT)

(56) References cited:
- WO-A-00/10582
- WO-A-96/31219
- US-A- 5 827 526
- GASKINS H R ET AL: "Dietary fructo-oligosaccharide modulates large intestinal inflammatory responses to Clostridium difficile in antibiotic-compromised mice." MICROBIAL ECOLOGY IN HEALTH AND DISEASE, vol. 9, no. 4, 1996, pages 157-166, XP000965229 ISSN: 0891-060X
- BEERS MH AND BERKOW R (EDS.): "The Merck Manual, 17th ed." 1999 , MERCK RESEARCH LABORATORIES, WHITEHOUSE STATION, NJ , ISBN: 0911910-10-7 XP002153983 * page 293 - page 295 * & "Antibiotic-associated colitis" * page 293 *

## Description

### Background of the invention

Antibiotics are very popular drugs to treat bacterial infection, but at the same time they are also frequently subjected to a large misuse, which contribute to the increase of reported side effects. Antibiotics may induce changes in the human gut flora as they are active against pathogen bacteria.

The gut flora plays an important role in the gastrointestinal homeostasis regulating the tone, motility, volume and intestinal gas. The integrity of gut flora is essential for vitamin biosynthesis, drug or xenobiotic metabolism, endogenous substances and hormones metabolism, pH regulation etc.

The antibiotic treatment may destroy the gut flora resulting in the exacerbation of gastrointestinal side effects; in this regard, many patients taking various oral penicillin preparations experience nausea, with or without vomiting, and some have mild to severe diarrhoea, often related to the dose of the drug. For instance, all the beta-lactam antibiotics, unrelated to the route of administration, change the composition of gut microbiota by eliminating the sensitive micro-organisms, which usually are re-established shortly, after antibiotic therapy is stopped. However, it is not unusual that superinfection results due to the changes in gut flora and pseudomembranous colitis may occur as overgrowth of Clostridium difficile (1).

Indeed, patients who have experienced gastrointestinal complaints during broad-spectrum antibiotic therapy may be reluctant to comply or re-start the therapy. It has been demonstrated that the administration of antibiotics with a diet supplement of yoghurt was associated with a significant decrease in patient complaints of gastrointestinal side effects and yeast superinfection (2). In this regard, several pharmaceutical products, already known also as probiotics, containing yeasts, enterobacteria, saccharomyces, bacillus subtilis, etc. are used during antibiotic therapy.

The use of large quantity of yoghurt, or exogenous supplied probiotic to reconstruct the endogenous microbiota may have some disadvantages such as: the yoghurt taste may be unpleasant to kids, and/or it is difficult to add an additional aliments specially to infants, the difficulty to supply an additional drug to young patients, and some-times even to elderlies. Moreover, although exogenously supplied prebiotics survive passage through the stomach and small intestine in human, they do not generally colonise the colon (3).

Fructans, also known as fructooligosaccharides or inulin, are natural substances composes primarily of fructose molecules characterised by their degree of polymerisation as already described in US patent N° 5,827,526. As used in this application, and in the present application and the claims, all the terms referable to fructans are interchangeable.
It is well known in the art that fructans selectively stimulate the growth of bifidobacteria (4) and therefore have been also named prebiotics which means "microbial food supplements that beneficially affect the host by improving its intestinal microbial balance" (5). Intake of fructans, increasing the growth of bifidobacteria at the expense of other bacteria maintains at low level clostridia or coliforms, which are potentially pathogen (6).

Moreover the use of fructans as prebiotic food has already been described in several documents such as:kkk
US patent N° 5,527,556 describe compositions having a creamy structure containing fructans for preparation of food products having improved nutritional properties, such as the proliferation of the useful intestinal flora.

Likewise, US patent N° 5,656,317 describe agglomerated composition comprising at least one fructan for producing food products to promote the development of a beneficial intestinal flora.

Likewise. US patent N° 5,840,361 describe fructan-containing baby food compositions to selectively stimulate colonic bifidobacteria in a human infant.

On the other hand, there are only few patents concerning the use or fructans in pharmaceutical preparations:

US patent N° 5,753,266 describe the use of safflower seed topical formulation which contains also fructans for the treatment of rheumatoid-based arthritic diseases and menopause.

Likewise US patent N° 5,593,658 describe biocompatible medical compositions comprising fructans as carrier for diagnostic agents.

### Aim of the invention

The present invention provides oral pharmaceutical formulations comprising one or more antibiotics at therapeutic dosages and fructans in a mixture of high- and low- polymerised fructans at prebiotic dosages, and used as excipients.

Sucrose is one of the most popular excipients used in oral pharmaceutical formulations including antibiotics; but, sucrose is a highly energetic compound and may require diet adjustment, and is contraindicated in diabetic patients, and in paediatric patients due to its cariogenic effect.

The use of fructans as pharmaceutical acceptable excipient overcomes all the limitation of sucrose, since fructans possess only modest energetic power, do not have cariogenic effect, and do not are contraindicated in diabetic patients. Moreover, fructans have prebiotic activity, which counteracts the flora gut destroying effect of antibiotics.

Several documents, as already mentioned, describe the prebiotic effect of fructan aliments, but recently US patent N°5,659,028 have reported the use of branched fructo-oligosaccharides in pharmaceutical compositions as, inter alia, bifidogenic agents; in addition these authors claim their use in pharmaceutical composition with a "pharmaceutically acceptable carrier" and filler", indicating that fructans are considered the active ingredient of the pharmaceutical preparation.

The same authors also stated that fructo-oligosaccharides "can be used as sugar substitute in standard application, for example as sweeteners in food and beverages or as base materials or excipients for the preparation of pharmaceutical products''.

Moreover WO 00/10582 discloses the use of therapeutic compositions comprising lactic-producing bacterial, antibiotics and more bifidogenic factors such as fructo-oligosaccharides (FOS), gluco-oligosaccharides (GOS), raffinose, and long-chain oligosaccharides. Again in this patent, as in the previous, the use of fructans has been disclosed only as active ingredients to obtain a prebiotic efficacy.

In the present invention fructans are used both as prebiotic and as excipients for oral formulations containing antibiotics, resulting in the lack or at least in a marked reduction of the need for additional pharmaceutical excipients, with an obvious practical advantage.

The present invention demonstrates additionally, that only a well defined mixtures of high- and tow-polymerised fructans are suitable to obtain oral liquid pharmaceutical formulation containing antibiotics and at the same time having the prebiotic dosage.

Indeed, to obtain solution/suspension pharmaceutically acceptable for an antibiotic it may be used high-polymerised fructans which have the rheological characteristics to form a structured vehicle. However, the maximal final concentration suitable for this use is too low to obtain an adequate prebiotic dosage. On the other hand using low-polymerised fructans alone it is easy to reach the prebiotic dosage, but whole text there is no disclosure of particular mixtures of low- and high- polymerised fructans, and therefore this patent application teaches that undefined mixture of low-and high- polymerised fructans may be used in pharmaceutical compositions.

On the contrary, and surprisingly we found that only particular and specific mixtures of low- and high- polymerised fructans are suitable for obtaining the wanted pharmaceutical compositions. In this regard, for example using high-polymerised fructans over 30% and low-polymerised fructans at about 10% to match the prebiotic dosage, the composition being too thick to homogenously suspend the antibiotic in a multidose formulation, such as reported in exemple 1. On the contrary, in similar experiments using high- and low-polymerised fructans at the concentrations of 10% and 30 % respectively, the capacity to suspend the antibiotic was too low.

Therefore, in the scope of the present invention, only using mixtures of high-and low-polymerised fructans in a well-defined range it is possible to obtain both a formulation with rheological characteristics pharmaceutically acceptable and to reach a prebiotic dosage.

### Summary of the invention

The present invention provides pharmaceutical oompositions comprising one or more antibiotics at therapeutic dosage and fructans, in a mixture of determined amounts of high- and low-polymerised fructans both as pharmaceutical acceptable excipients and sufficient for the prebiotic dosage.

Antibiotics, all of which are believed to be useful in the present invention, are an art-recognised class of drugs that possesses antimicrobical activity. They can generally be grouped into different chemical classifications:

Beta-lattam including penicillins with broad spectrum such as: ampicillin, amoxicillin, bacampicillin, phenoxymethylpenicillin, dicloxacillin, fludoxacillin, their combinations, and pharmaceutical acceptable salts thereof. Combinations of the above-mentioned antibiotics include also clavulanic acid.

Cephalosporins such as: cephalexin, cefuroxime, cefaclor, cefadroxil, cefatrizine, cefixime, cefetamet, cephradine, cefpodoxime, loracarbef, ceftibuten, their combinations, and pharmaceutical acceptable safts thereof.

Tetracyclines such as: dimechlocycline, doxycycline, chlortetracycline, methacycline, tetracycline, minocycline, their combinations, and pharmaceutical acceptable salts thereof.

Amphenicols such as: chloramphenicol, tiamphenicol, their combinations, and pharmaceutical acceptable salts thereof.

Sulfonamides and trimethoprim such as: trimethoprim, brodimoprim, sulfadiazine, sulfadimethoxine, sulfalene, sulfamethoxazole, sulfametrole, their combinations, and pharmaceutical acceptable salts thereof.

Macrolides such as: erythromycin, spiramycin, midecamycin, roxithromycin, josamycin, throleandomycin, clarithromycina, azithromycin, miocamycin, rokitamycina, dirithromycina, flurythromicina, clindamycin, lincomycin, their combinations, and pharmaceutical acceptable salts thereof.

Quinolones such as: ofloxacin, ciprofloxacin, pefloxaan, enoxacin, norfloxacin, lomefloxacin, rufloxacin, and their combinations.

Preferred antibiotics for use in the combination of the invention include also antibiotics of the different class if they are pharmaceutically compatible.

Preferred fructans for use in the present invention have a polymerised degree ranging from 2 to about 60.

Preferred mixtures of fructans for use in the present invention include low-polymerised fructans having a degree of polymerisation (DP) ranging from 2 to 6, and most preferred from 2 to 4, and high-polymerised fructans having a degree of polymerisation ranging from 7 to about 60 and most preferred from 8 to 30.

The ratio between high- and low-polymerised fructans for use in the present invention ranges from 0.3 to 1, to 3 to 1 by weight; most preferable are mixtures containing low-polymerised fructans ranging from about 10 to about 40 % by weight of the final formulation, and high-polymerised fructans ranging from about 10 to about 65% by weight, and most preferable ranging from about 15 to about 55 % by weight of the final formulation.

The preferred prebiotic daily dosage of fructans for use in the present invention ranging from about 2 to about 20 g/die, and most preferable from about 3 to about 9 g/die, which may be divided in different administrations to obtain the final total amount, following the requirement of the antibiotic dosage regimen.

Preferred pharmaceutical formulations for use in the present invention include forms suitable for oral administration which may be presented as discrete unit dosage forms such as: tablets, hard or soft gelatin capsules, cachets; as a powder or as granules; as a solution or suspension or as an emulsion; or in a chewable base; each containing a predertermined amount of the antibiotic and fructans. These formulations may also contain conventional excipients and other ingredients conventionally used by a person skilled in the art.

Particularly preferred oral pharmaceutical formulations for use in the present invention are oral liquid preparations, both as discrete unit dosage or suitable for multiple divided dosage, which may be in the form of, for example, aqueous or oily suspensions, solutions, emulsions, syrups or elixir, or may be presented as dry product for reconstitution with water or other suitable vehide before use. Such preparations may also contain conventional excipients and other ingredients conventionally used to a person skilled in the art.

### EXAMPLES

Processes used and the products obtained are better described referring to the following examples, which have to be considered explanatory and not restrictive of the invention. It is intended that variations and modifications can be effected within the spirit and the scope of the present invention.

### Example 1

### Preparation of a reconstituted suspension of amoxiallin.

The powder for the suspension can be prepared mixing 5.74 g amoxicillin trihydrate, 18 g high-polymerised fructans, 22 g low-polymerised fructans and 0.5 saccharinate sodium. The suspension is obtained adding water for reconstitution (up to 100 ml) and after vigorous shaking it remains homogeneous without flocculation at least 48 hours also at 4°C temperature.

If necessary, dyes and flavourings will be added to improve the organoleptic properties. In the present example there was no need to add any other ingredients, however, during the industrial phase glidants, wetting agents, anti-foaming agents or other pharmaceutically acceptable inactive ingredients could be added to improve the productive process.

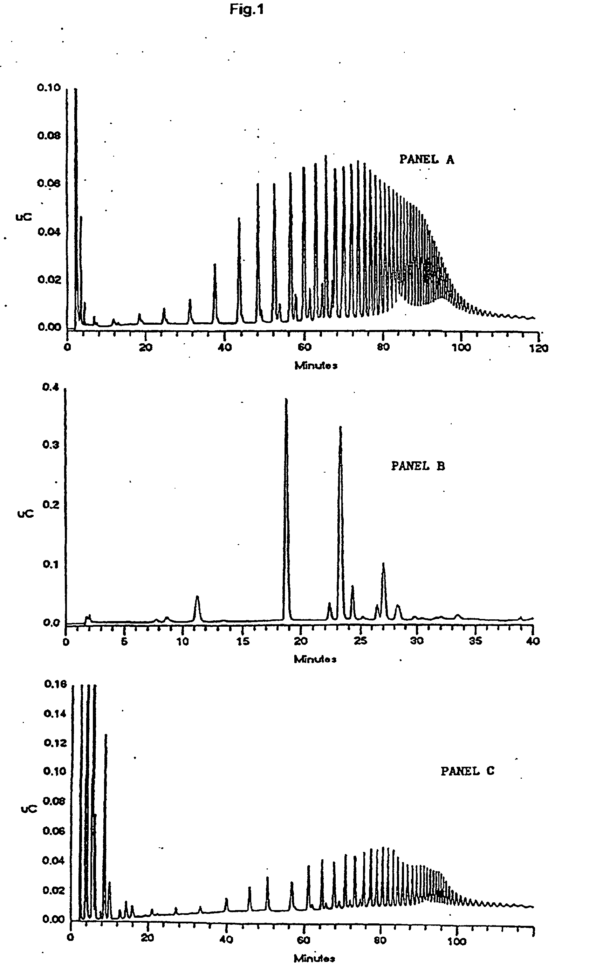

A chromatogram of the fructans and mixture of fructans used is shown in fig. 1: panel A high-polymerised fructans, panel B low-polymerised fructans; panel C a mixture in the ratio of 1:3 of low- and high-polymerised fructans respectively.

Separation and characterization of high- and low-polymerised fructans have been carried out by HPLC analysis using the Dionex Bio-LC 4000i gradient pump module equipped with a Pulsed electrochemical detector (PED-2). Detection of high- and low-polymerised fructans has been performed in the pulsed amperometric detection mode (PAD), with a gold working electrode and a silver-silver chloride reference electrode. The electrode pulse potentials and duration were as those reported elsewhere (7). Separations have been performed at room temperature on a CarboPac PA-100 analytical column (4 x 250 mm) connected to a CarboPac PA-100 guard column. High-and low-polymerised fructans have been eluted by a suitable gradient program increasing sodium acetate in the mobile phase consisting of 90 mM sodium hydroxide at flow rate of 0.8 ml /min.

### Example 2

### Preparation of reconstituted powder of amoxicillin and clavulanate potassium.

The formulate can be obtained mixing 1004.5 mg amoxicillin trihydrate, 148.9 mg clavulanate potassium, 4 g high-polymerised fructans, 2 g low polymerised-fructans, 0.4 g saccharinate sodium. The mixture is easily dispersible in about a hatf glass of water or any other edible liquid.

### Example 3

### Preparation of a reconstituted suspension of cefaclor.

The granulate for the suspension can be prepared using 5.26 g cefaclor monohydrate, 18 g high-polymerised fructans, 22 g low-polymerised fructans and 0.5 g saccharinate sodium. The suspension is obtained adding water for reconstitution (up to 100 ml) and after vigorous shaking it remains homogeneous without flocculation at least 48 hours also at 4°C temperature.
If necessary, dyes and flavourings will be added to improve the organoleptic properties. In the present example there was no need to add any other ingredients, however, during the industrial phase glidants, wetting agents, anti-foaming agents or other pharmaceutically acceptable inactive ingredients could be added to improve the productive process.

### Example 4

### Preparation of a reconstituted suspension of clarithromycin.

The granulate for the suspension can be prepared using 2,5 g clarithromycin, 18 g high-polymerised fructans, 22 g low-polymerised fructans and 0.7 saccharinate sodium. The suspension is obtained adding water for reconstitution (up to 100 ml) and after vigorous shaking it remains homogeneous without apparent flocculation.
If necessary, dyes and flavourings will be added to improve the organoleptic properties. In the present example there was no need to add any other ingredients, however, during the industrial phase glidants, wetting agents, anti-foaming agents or other pharmaceutically acceptable inactive ingredients could be added to improve the productive process.

### Example 5

### Preparation of reconstituted powder of erythromycin.

The formulate can be obtained mixing 1 g erythromycin, 4 g high-polymerised fructans, 0.8 g saccharinate sodium. The mixture is easily dispersible in about hatf glass of water or any other edible liquid.

### REFERENCES

1) Goodman and Gilman's the pharmacological basis of therapeutics, 8^{th} edition, Pergamon Press.
2) Witsell DL. et al. J. Otolaryngol. 1995; 24(4): 230-3 (abstract only)
3) Bengmark S. Nutrition 1998; 14: 585-94
4) Roberfroid MB. Adv. Exp. Med. Biol. 1997; 427: 211-9 (abstract only)
5) Gibson GR. & Roberfroid MB. J. Nutr. 1995; 125(6): 1401-12 (abstract only)
6) Reddy BS. et al. Caranogenesis 1997; 18(7): 1371-4
7) Corradini C. et al. Sem in Food Sci 1997; 2: 99-111

## Claims

1. Pharmaceutical oral compositions comprising one or more antibiotics in therapeutically effective amount and, as main excipient, a mixture of high- and low-polymerised fructans in the ratio from 0.3 to 1, to 3 to 1 by weight in prebiotic effective amount.

2. Pharmaceutical oral compositions according to claim 1 wherein low-polymerised fructans have a degree of polymerisation comprised from 2 to 6 DP, and most preferred from 2 to 4 DP, and high- polymerised fructans is comprised from 7 to 60 DP and most preferred from 8 to 30 DP.

3. Pharmaceutical oral compositions according to claims 1 and 2 wherein the ratio between high- and low-polymerised fructans most preferable are the mixtures containing low-polymerised fructans ranging from 10 to 40 % by weight of the final formulation, and high-polymerised fructans ranging from 10 to 65% by weight, and most preferable ranging from 15 to 55 % by weight of the final formulation.

4. Pharmaceutical oral compositions according to claims from 1 to 3 wherein the formulations or a fraction thereof allow a dosage of antibiotic effective for achieving therapeutic activity and a daily amount of fructans at prebiotic dosage, and comprised from 1 to 20 g/day, being most preferred from 3 to 9 g/day.

5. Pharmaceutical oral compositions according to claims from 1 to 4 wherein said antibiotics at therapeutic dosage are selected in the groups consisting of beta-lactam antibiotics, cephalosporins, tetracyclines, amphenicols, sulphonamides and trimethoprim, macrolides, quinolones antibiotics.
• Beta-lactam antibiotics comprise, penicillins: ampicillin, amoxicillin, bacampicillin, phenoxymethylpenicillin, dicloxacillin, flucloxacillin , associations thereof and associations with clavulanic acid or its salts;
cephalosporins: cefalexin, cefuroxime, cefaclor, cefadroxil, cefatrizine, cefixime, cefetamet, cefradine, cefpodoxime, loracarbef, ceftibuten, and their combinations, including clavulanic acid, and pharmaceutical acceptable salts thereof.
• Tetracyclines comprise demeclocycline, doxycycline, chlortetracycline, methacycline, tetracycline, minocycline, and their combinations, and pharmaceutical acceptable salts thereof.
• Amphenicols comprise chloramphenicol, thiamphenicol and associations thereof.
• Sulphonamides and trimethoprim comprise sulfadiazine, sulfdadimethoxine, sulfalene, sulfamethoxazole, sulfametrole trimethoprim, brodimoprim, and their combinations, and pharmaceutical acceptable salts thereof.
• Macrolides comprise erythromycin, spiramycin, midecamycin, roxithromycin, josamycin, troleandromycin, clarithromycin, azithromycin, miocamycin, rokitamycin, dirithromycin, flurithromycin, clindamycin, lincomycin, and their combinations, and pharmaceutical acceptable salts thereof.
• Quinolone antibiotics comprise ofloxacin, ciprofloxacin, pefloxacin, enoxacin, norfloxacin, lomefloxacin, rufloxacin, and their combinations, and pharmaceutical acceptable salts thereof.
Combinations among antibiotics of different groups are comprised provided that pharmaceutically acceptable.

6. Pharmaceutical compositions according to claims from 1 to 5 in forms adapted for oral administration being most preferred oral liquid formulations both as discrete unit dosage or multiple divided dosage.

## Patentansprüche

1. Pharmazeutiche Zusammensetzungen für die orale Verabreichung, die ein oder mehrere Antibiotika in therapeutisch wirksamer Menge und, als wichtigsten Hilfstoff, eine Mischung hoch- und geringpolymerisierter Fruktanen im Bereich von 0.3 bis 1 Gewichtsprozent, 3 bis 1 Gewichtsprozent in prebiotik wirksamer Menge, enthalten.

2. Pharmazeutiche Zusammensetzungen für die orale Verabreichung nach Anspruch 1, wobei das Polymerisationsgrad für geringpolymerisierte Fruktane im Bereich von 2 bis 6 DP, vorzugsweise von 2 bis 4 DP, und für hochpolymerisierte Fruktane im Bereich von 7 bis 60 DP, vorzugsweise von 8 bis 30 DP liegt.

3. Pharmazeutiche Zusammensetzungen für die orale Verabreichung nach Ansprüchen 1 und 2, wobei Verhältnisse hoch- und geringpolymerisierter Fruktanen Mischungen, die geringpolymerisierte Fruktane im Bereich von 10 bis 40% Gewichtsprozent der Endformulation und hochpolymerisierte Fruktane im Bereich von 10 bis 65% Gewichtsprozent, vorzugsweise im Bereich von 15 bis 55% Gewichtsprozent der Endformulation enthalten, bevorzugt sind.

4. Pharmazeutische Zusammensetzungen für die orale Verabreichung nach Ansprüche 1 - 3, wobei die Formulierungen oder ein Teil davon eine Dosierung von Antibiotika ermöglichen, mit der eine therapeutische Aktivität erlangt wird und eine tägliche Dosierung an Fruktanen in prebiotiker Menge im Bereich von 1 bis 20 g/Tag, vorzugsweise von 3 bis 9 g/Tag.

5. Pharmazeutiche Zusammensetzungen für die orale Verabreichung nach Ansprüchen 1 - 4, wobei die obengenannten Antibiotika in therapeutischer Menge unter Beta-Laktam-Antibiotika, Cephalosporine, Tetracycline, Amphenicole, Sulphonamiden und Trimethoprim, Makrolide, Quinolinone Antibiotika ausgewählt sind.
• Beta-Laktam-Antibiotika umfassen:
Penicilline: Ampicillin, Amoxicillin, Bacampicillin, Phenoxymethylpenicillin, Dicloxacillin, Flucloxacillin, ihre Verbindungen und Verbindungen mit Clavulaninsäure oder ihre Sälze;
Cephalosporine: Cefalexin, Cefuroxim, Cefactor, Cefadroxil, Cefatrizin, Cefixim, Cefetamet, Cefradin, Cefpodoxim, Loracarbef, Ceftibuten und ihre Verbindungen, einschließlich Clavulaninsäure, und ihre pharmazeutisch akzeptabeie Sälze.
• Tetracycline umfassen Demeclocyclin, Doxycyclin, Chlortetracyclin, Metacyclin, Tetracyclin, Minocyclin, ihre Verbindungen und ihre pharmazeutisch akzeptabele Sälze.
• Amphenicole umfassen: Chloramphenicol, Triamphenicol und ihre Verbindungen.
• Sulfonamide und Trimethoprim umfassen: Sulfadiazin, Sulfadimethoxin, Sulfalen, Sulfamethoxazol, Sulfametrol Trimethoprim, Brodimoprim, ihre Verbindungen und ihre pharmazeutisch akzeptabele Sälze.
• Makrolide umfassen Erythromycin, Spiramycin, Midecamycin, Roxithromycin, Josamycin, Troleandromycin, Clarithromycin, Azithromycin, Miocamycin, Rokitamycin, Dirithromycin, Flurithromycin, Clindamycin, Lincomycin, ihre Verbindungen und ihre pharmazeutisch akzeptabele Sälze.
• Quinolone Antibiotika umfassen: Ofloxacin, Ciprofloxacin, Pefioxacin, Enoxacin, Norfloxacin, Lomefloxacin, Rufloxacin, ihre Verbindungen und ihre pharmazeutisch akzeptabele Sälze.
Kombinationen von Antibiotika der verschiedenen Gruppen, unter der Bedingung, daß dieselben pharmazeutisch akzeptabel sind.

6. Pharmazeutische Zusammensetzungen nach Ansprüchen 1 bis 5 in geeigneter Form für die orale Verabreichung, wobei orale flüssige Formulierungen mit Einzeln-oder Mehrfachdosierung bevorzugt sind.

## Revendications

1. Formulations pharmaceutiques orales contenant un ou plus antibiotiques in quantité thérapeutique efficace et, comme majeur excipient un mélange de fructo-oligosaccharides à haute et basse polymérisation en rapport de poids allant de 0.3 - 1 jusqu'à 3 - 1 avec une quantité efficace prébiotique.

2. Formulations pharmaceutiques orales selon la revendication 1, où les fructo-oligosaccharides à basse poymérisation ont un degré de polymérisation compris entre 2 et 6 DP, et plus préférable entre 2 et 4 DP, et de fructo-oligosaccharides à haute polymérisation comprise entre 7 et 60 DP, et plus préférable entre 8 et 30 DP.

3. Formulations pharmaceutiques orales selon la revendication 1 et 2 où les rapports les plus préférables parmi les fructo-oligosaccharides à haute et basse polymérisation, sont les mélanges contenant les fructo-oligosaccharides à basse polymérisation allant de 10 à 40 % en poids de la formulation finale, et les fructo-oligosaccharides à haute polymérisation allant de 10 à 65 % en poids et plus préférable entre15 à 55 % en poids de la formulation finale.

4. Formulations pharmaceutiques orales selon les revendications allant de 1 à 3 où la formulation, ou en part, permet une dose d'antibiotique suffisante à avoir une activité thérapeutique et une quantité journalière de fructo-oligosaccharides avec un dosage pré-biotique allant de 1 à 20 g/ jour, et plus préférable allant de 3 à 9 g/ jour.

5. Formulations pharmaceutiques orales selon les revendications allant de 1 à 4 où les antibiotiques en dosage thérapeutique sont sélectionnés dans le groupe des antibiotiques: Beta-Lactams, Cephalosporins, Tetracyclines, Amphenicols, Sulphonamides Et Trimethoprim, Macrolides, Quinolones Antibiotiques.
• Antibiotiques beta-lactams comprenant
Penicillins: Ampicillin, Amoxicillin, Bacampicillin, Phenoxymethylpenicillin, Dicloxacillin, Flucloxacillin, leurs melanges et melanges avec Clavulanic acid ou ses sels;
Cephatosporins: Cefalexin, Cefuroxime, Cefaclor, Cefadroxil, Cefatrizine, Cefixime, Cefetamet, Cefradine, Cefpodoxime, Loracarbef, Ceftibuten, et leurs melanges, comprenant Clavulanic acid, et leurs sels pharmaceutiquement acceptables.
• Tetracyclines comprenant Demeclocycline, Doxycycline, Chlortetracycline, Methacycline, Tetracycline, Minocycline, et leurs melanges et leurs sels pharmaceutiquement acceptables.
• Amphenicols comprenant Chloramphenicol, Thiamphenicol et leurs mélanges.
• Sulphonamides et trimethoprim comprenant sulfadiazine, sulfdadimethoxine, sulfalene, sulfamethoxazole, sulfametrole trimethoprim, brodimoprim, et leurs melanges et leurs sels pharmaceutiquement acceptables.
• Macrolides comprenant Erythromycin, Spiramycin, Midecamycin, Roxithromycin, Josamycin, Troleandromycin, Clarithromycin, Azithromycin, Miocamycin, Rokitamycin, Dirithromycin, Flurithromycin, Clindamycin, Lincomycin, et leurs melanges et leurs sels pharmaceutiquement acceptables.
• Quinolone antibiotiques comprenant Ofloxacin, Ciprofloxacin, Pefloxacin, Enoxacin, Norfloxacin, Lomefloxacin, Rufloxacin, et leurs melanges et leurs sels pharmaceutiquement acceptables.
Les mélanges avec antibiotiques de groupes différents sont compris si pharmaceutiquement acceptables.

6. Formulations pharmaceutiques selon les revendications allant de 1 à 5 sous formes aptes à l'administration orale et les plus préferables sont les formulations liquides orales aussi bien pour un seul dosage que pour un dosage multipte.
